# EUROPEAN PATENT SPECIFICATION

(11) **EP 1 154 734 B1**
(45) Date of publication and mention of the grant of the patent: **01.10.2014**
(21) Application number: 00917614.0
(22) Date of filing: 07.02.2000
(51) Int. Cl.: A61L 27/24, A61L 27/38, A61L 27/54, A61K 47/42, A61K 31/522, A61K 35/12, A61K 9/00

(54) **INJECTABLE COLLAGEN-BASED SYSTEM FOR DELIVERY OF CELLS**
INJIZIERBARES KOLLAGEN ZUR VERABREICHUNG VON ZELLEN
SYSTEME INJECTABLE A BASE DE COLLAGENE POUR L'ADMINISTRATION DE CELLULES

(30) Priority: 12.02.1999 US 119981 P
(43) Date of publication of application: 21.11.2001
(73) Proprietor: ProChon Biotech Ltd., 76114 Rehovot (IL)
(72) Inventor: DEVORE, Dale, P., Chelmsford, MA 01824 (US)
(74) Representative: Becker Kurig Straus
(86) International application number: PCT/US2000/003117
(87) International publication number: WO 2000/047130

(56) References cited:
- EP-A- 0 671 165
- US-A- 5 550 188
- US-A- 5 660 692
- US-A- 5 800 541
- YOUNG R G ET AL: "USE OF MESENCHYMAL STEM CELLS IN A COLLAGEN MATRIX FOR ACHILLES TENDON REPAIR" JOURNAL OF ORTHOPAEDIC RESEARCH, JOHN WILEY & SONS, INC, NEEDHAM, MA, vol. 16, no. 4, 1 July 1998 (1998-07-01), pages 406-413, XP008005059 ISSN: 0736-0266
- FUJIOKA K ET AL: "Protein release from collagen matrices", ADVANCED DRUG DELIVERY REVIEWS, ELSEVIER BV, AMSTERDAM, NL, vol. 31, no. 3, 4 May 1998 (1998-05-04), pages 247-266, XP002250166, ISSN: 0169-409X, DOI: 10.1016/S0169-409X(97)00119-1

## Description

### Background of the Invention

In general, this invention relates to a collagen delivery system to administer a therapeutic agent such as a cell preparation, a growth factor, a steroid, an antibiotic, or a regulator of angiogenesis or cell proliferation.

Numerous properties of collagen favor its use as a biological delivery agent (Simpson, Biomaterials in Reconstructive Surgery, In Collagen as a Biomaterial, C.V. Mosby Co.,Ch. 11, pages 109-117, 1983; DeVore, Collagen as an Opthalmic Biomaterial, In Encyclopedic Handbook of Biomaterials & Bioengineering, Part B, Applications, Marcel Dekker, Inc., New York, Ch. 45, 1995; Silver and Garg, Collagen: Characterization, Processing and Medical Applications, In Handbook of Biodegradable Polymers, Eds. Domb, Kost, and Wiseman, Harwood Academic Publishing, Australia, Ch. 17).

For example, one can design collagen products in which the time required for resorption ranges from a few minutes to months. This rate of collagen resorption is controlled by the form of collagen extracted or prepared and by the degree of collagen crosslinking. In particular, collagen molecules with stabilized, reducible, intramolecular and intermolecular crosslinks take the form of insoluble, condensed collagen fibrils that are resorbed slowly in vivo. In contrast, collagen molecules with few, if any, crosslinks are soluble in aqueous salt, acidic solutions, and basic solutions, and are quickly resorbed. Therefore, one can prepare collagen in various forms or can chemically modify collagen to obtain formulations of the desired resorption time while still retaining collagen's hydrophilic and non-immunogenic properties.

The ability to specifically deliver a compound to a particular site in the human body is a desirable goal in many areas of medicine. Such delivery (especially by injection) is particularly beneficial, for example, to accurately administer a sufficient dose to the site of interest while, at the same time, minimizing exposure to surrounding tissues. Site specific delivery is beneficial, for example, to enhance bone repair (Miyamoto and Takaoka, Ann. Chir. Gynaecol. Suppl. 207:69-75, 1993), cartilage repair (Buckwalter and Mankin, Instr. Course Lect. 47: 487-504, 1998), tendon repair (Young et al., J. Orthop. Res. 16: 406-413, 1998), wound healing (Rao, J. Biomater. Sci. Polymer Edn. 7: 623-645, 1995), and peripheral nerve regeneration (Seckel et al., J. Neurosci. Res. 40: 318-324, 1995), as well as to prevent local infection (Wachol et al., Biomaterials 17: 1733-1738, 1996; Gebhardt and Kaufman, J. Ocul. Pharmacol. Ther. 11 : 319-327, 1995), cellular proliferation (Kay et al., Ophthalmic Surg. 17: 796-801,1996), and local tumor growth (Davidson et al., J. Surg. Res. 58: 618-624, 1995)

The remodeling of the adult skeleton involves the ongoing processes of bone formation and resorption as well as the regulation of serum calcium. Following bone injury, significant numbers of osteoprogenitor cells can be mobilized at the defect site to produce large quantities of bone matrix. In addition, mesenchymal cells migrate from the marrow and differentiate into chondroblasts that produce a cartilagenous extracellular matrix, subsequently replaced by bone. These processes are controlled, at least in part, by the large number of growth factors present in the bone matrix after injury (Wozney and Rosen, Handbook of Experimental Pharmacology, In Physiology and Pharmacology of Bone, Eds. Mundy and Martin, Springer Verlag, Berlin, Vol. 107, 1993).

The cartilage injury response differs from other tissues because of the relative avascularity of cartilage and the limited chondrocyte mobility and proliferation in cartilage (Newman, Am. J. Sports Med. 20: 309-324, 1998). In other tissues, by contrast, the initial stage of wound healing is characterized by the movement of intravascular components, such as plasma and blood proteins, to the extravascular area (Peacock, In Wound Repair, Ed. Peacock, WB Saunders Co, Philadelphia, PA, pages 491-492, 1984). Neutrophils and macrophages then migrate to the injury site, functioning to prevent infection and promote fibroblast migration. Subsequent phases of wound healing include fibroblast secretion of collagen, collagen stabilization, angiogenesis, and wound closure (Costa et al., Opth. Surgery 24: 152-170, 1993).

EP 0 071 165 discloses a collagen-based injectable drug delivery system and its use to achieve delivery of drug to a patient at a sustained rate over a prolonged time period. In particular, the system includes collagen, a cross-linking agent capable of forming covalent bonds in situ, following injection with the collagen, a flexible chain polymer similar in charge to the collagen, and a drug in a pharmaceutically acceptable injectable carrier.

Young RG et al. ("Use of mesenchymal stem cells in a collagen matrix for Achilles tendon repair"; Journal of Orthopedic Research, John Wiley & Sons, Inc., Needham, MA, Vol. 16, No. 4, July 1, 1998, pages 406-413) reports on the use of mesenchymal stem cells in a collagen matrix for achilles trendon repair. To this end, a collagen gel without crosslinkers is suggested, which can be used to affix mesenchymal stem cells to a surgical suture. It is deemed necessary to incubate the solution for 40 hours at 37°C prior to use. Young et al. teach to implant the collagen solution after the gel has formed, and in conjunction with a separate, non-collagen construct. Thus, Young et al. suggest an already polymerized matrix comprising mesenchymal stem cells.

US 5,800,541 discloses collagen-synthetic polymer matrices. A collagen is reacted with a functionally activated synthetic hydrophilic polymer to form a collagen-synthetic polymer matrix which is further modified by crosslinking, conjugating or coupling. Potential coupling partners are described as biologically active molecules such as growth factors, cytokines and active peptides.

### Summary of the Invention

In general, the invention features delivery of a therapeutic agent to a tissue site, involving:
(a) combining the therapeutic agent with a soluble collagen to form a therapeutic agent-collagen solution; and
(b) administering the solution to the tissue site whereby, upon contact with the tissue, the solution is converted to a gel and the therapeutic agent is delivered to the site.

Thus, according to a first aspect of the present invention, there is provided an initially soluble collagen capable of rapid polymerization for use as a vehicle for delivering stem cells. Said stem cells are combined with the soluble collagen to form a stem cell-collagen solution which after administration to a tissue site and upon contact with the tissue rapidly polymerizes to a gel, thereby delivering the stem cells to the tissue site. The collagen solution further comprises a fibrillar collagen component, and upon contact with a tissue the solution is converted to a gel within time intervals selected from the group of 180 seconds, 120 seconds, and 90 seconds. The fibrillar collagen component is at a concentration of between 0.01-2.0% fibrillar collagen solids (w/v).

According to one embodiment, the stem cells can be mesenchymal stem cells.

According to another embodiment, the solution can be injectable.

According to one preferred embodiment, the tissue site is bone, cartilage, meniscus, tendon, ligament, or dermis.

According to another embodiment, the tissue site has a defect. Preferably, said defect is treated by said delivering of said stem cells to said tissue site.

The duration of delivering of said therapeutic agent or said cells can be extended by crosslinking said therapeutic agent to said collagen in said solution or by increasing the crosslinking of said collagen in said solution. Preferably, said crosslinking is induced chemically or by UV radiation.

According to another aspect of the present invention, there is provided a stem cell-collagen solution capable of rapidly polymerizing to a collagen gel upon contact with a tissue, wherein the collagen solution further comprises a fibrillar collagen component, wherein upon contact with a tissue said solution is converted to a collagen gel within time intervals selected from the group of 180 seconds, 120 seconds, and 90 seconds, and wherein said fibrillar component is at a concentration of between 0.01-2.0% fibrillar collagen solids (w/v).

According to one embodiment, the stem cells are mesenchymal stem cells.

According to another embodiment, the solution is injectable.

According to yet another embodiment, the stem cells are crosslinked to said collagen in said solution. Preferably, the collagen is crosslinked in said solution.

The crosslinking can be induced chemically or by UV radiation.

By "tissue" is meant an aggregation of similarly specialized cells in an organism, preferably, mammalian, and, most preferably, human, where the cells are exposed to the organism's extracellular fluid, and are united in performance of a function within an organism.

By "therapeutic agent" is meant a substance that is administered to improve the condition of a tissue following injury or as a result of disease or degeneration.

By "fibrillar component" is meant an insoluble fibrillar collagen component wherein the collagen molecules interact in a quarter-stagger array to form microfibrils which themselves aggregate by side-to-side and end-to-end association to form stabilized collagen fibrils. The fibrillar component exhibits a collagen solid content ranging from about 0.1-2.0% (w/v) fibrillar collagen solids.

By "fibrillar collagen solids" is meant the dry collagen solid content of the fibrillar component in terms of percent fibrillar solids. Prior to addition to a soluble collagen formulation, the collagen solids are suspended in solution at a concentration ranging from 10-100 mg/ml, preferably 40 mg/ml. The collagen solid suspension is then added to the soluble collagen formulation for a final collagen solid concentration of 0.1-2.0%, or 1-20 mg/ml.

The present invention provides a number of advantages. For example, the collagen delivery systems described herein are biocompatible, readily available, biodegradable, and stable in solution at neutral pH. The ability to manipulate the collagen in solution form allows for the homogeneous dispersion of the therapeutic agents and the injectable administration of these agents through a fine gauge needle (e.g., a 30 gauge needle). In addition to the ease of application, injectable delivery also allows access to the administration site while minimizing invasive injury to surrounding tissues. The density of the collagen solution is sufficient to fill a bone defect or other specific delivery site and remain in place until gelation and fibril formation occurs, as well as to maintain the active therapeutic agent in the injection site for a period of time sufficient to promote the effect.

The disclosed collagen delivery system also has the advantage of allowing for the adjustment of therapeutic agent concentrations to achieve the desired dose. Furthermore, the crosslinking of molecules in the therapeutic agent-collagen formulation can be adjusted to extend the duration of therapeutic agent release. After polymerization, the injectable collagen delivery system also provides a scaffold-like structure to support new tissue growth.

In addition to the above, the collagen delivery system also has the advantage of providing local delivery. Thus, when the therapeutic agent has toxic side effects, e.g., chemotherapeutic agents, administration to a specific injection site minimizes exposure to s rounding tissues and dramatically reduces the undesirable, unnecessary side effects caused to the surrounding tissues or the body as a whole, while facilitating delivery of potent doses to the targeted cells. The local delivery system further has the advantage of minimizing the total amount of therapeutic agent required, and thus the cost of treatment.

Other features and advantages of the invention will be apparent from the detailed description thereof and from the claims.

### Description of the Drawing

FIGS. 1A (4X magnification) and IB (20X magnification) are photographs of an MSC-collagen gel maintained in culture for 7 days and stained with toluidine blue. No extracellular matrix formed.
FIGS. 2A (4X magnification) and 2B (20X magnification) are photographs of an MSC-collagen gel, cultured in vitro for 7 days, implanted subcutaneously in nude mice for 3 weeks, and then removed and stained with toluidine blue. An extracellular matrix with chondrocyte-like cells was observed as well as chondrocyte hypertrophy and endochondral bone formation.
FIG. 3 (20X magnification) is a photograph of an MSC-collagen gel, initially injected subcutaneously in nude mice as a solution which subsequently polymerized. The gel was removed 6 weeks post-injection and stained with toluidine blue. Pronounced extracellular matrix formation was observed, including chondrogenesis, osteogenesis, and the formation of openings resembling marrow space.
FIGS. 4A-4C are a series of photographs showing the gelation of the collagen solution upon exposure to PBS. FIG. 4A shows the collagen solution prior to addition to buffer. FIG. 4B shows gelation of demeclocycline-collagen upon exposure to PBS. FIG. 4C shows the demeclocycline-collagen gel after one hour in buffer.
FIG. 5 is a chart showing cumulative fluorescamine release from collagen gel over time.
FIG. 6 is a chart showing cumulative demeclocycline release from a collagen gel over time.
FIG. 7 is a graph describing the rate of fluorescamine release from a collagen gel. At 54 hours the percent release increased to 92.1%.
FIG. 8 is a graph describing the rate of demeclocycline release from a collagen gel.
FIG. 9 shows the spectrophotometric absorbance of acyclovir in saline.
FIG. 10 shows the spectrophotometric absorbance of a rabbit fibrillar collagen fraction crosslinked to acyclovir.
FIG. 11 shows the spectrophotometric absorbance of a human fibrillar collagen fraction (1:10 dilution) crosslinked to acyclovir. The analysis was conducted 8 days after treatment with 0.02% trypsin.
FIG. 12 shows the spectrophotometric absorbance of the first PBS wash (1 :10 dilution) following trypsin treatment of the human fibrillar collagen fraction described in FIG. 11.
FIG. 13 shows the spectrophotometric absorbance of the second PBS wash following trypsin treatment of the human fibrillar collagen fraction described in FIG. 6.
FIG. 14 shows the spectrophotometric analysis of the human fibrillar fraction crosslinked to acyclovir following digestion with collagenase.
FIG. 15 shows the spectrophotometric analysis of an acyclovir-free human fibrillar fraction following digestion with collagenase.
FIG. 16 shows the spectrophotometric analysis of crosslinked acyclovir released from collagen-based gels into the supernatant from 1 to 48 hours.
FIG. 17 is a graph of crosslinked acyclovir released from collagen-based gels (absorbance) into the supernatant as a function of time.
FIG. 18 shows the spectrophotometric analysis of crosslinked acyclovir released from collagen-based film from 2 hours to 6 days.
FIG. 19 is a graph depicting the release of crosslinked acyclovir from collagen-based film into the supernatant as a function of time.

### Detailed Description

Described herein is a delivery system which uses an initially soluble collagen capable of rapid polymerization as a vehicle for delivery of a therapeutic agent, for example, a cell preparation or therapeutic peptide. The low viscosity of the initial soluble collagen allows homogeneous mixing with the therapeutic agent and administration to a site, for example, by injection. The rapid polymerization characteristic of the collagen vehicle allows for targeted delivery and maintenance of the therapeutic agent at specific tissue sites. In addition, the dose and rate of delivery may be readily controlled by regulation of the concentration and in vivo release kinetics of the therapeutic agent.

Any collagen which exhibits the properties of an initially soluble state followed by rapid polymerization (preferably, within 180 seconds) may be used in the invention. Because this delivery system is optimally designed for use in mammalian systems, rapid polymerization preferably occurs at temperatures and pH's which approximate the physiological conditions of the recipient mammal. For humans, this collagen preferably polymerizes in a temperature range between 36°-39°C and at a pH range between 6.5-7.5.

Particularly preferred collagens for use in the invention are described in DeVore & Eiferman (U.S. Patent No. 5,492,135.) These collagens are initially soluble in form and, upon exposure to physiological fluids in vivo, undergo rapid polymerization. Such collagen solutions have been prepared at concentrations ranging from 10 mg/ml to over 70 mg/ml and at pH's ranging from 6.0-8.0.

The collagen systems described herein may be used to deliver any therapeutic agent or pharmaceutical agent of appropriate solubility
characteristics, including, without limitation, a cell preparation (wherein the cells themselves are therapeutic, e.g., a mesenchymal stem cell preparation or transfected cells which produce a therapeutic peptide), a growth factor, a steroid, an antibiotic, an angiogenic agent, an inhibitor of angiogenesis, and an inhibitor of cell proliferation. In each case, the therapeutic agent is added to the collagen solution to a desired concentration, and the solution is administered to the appropriate tissue site. The therapeutic agent can be entrapped in the collagen, or covalently bound and crosslinked to the collagen. Combinations of two or more therapeutic agents may be administered simultaneously, or the therapeutic agent may be combined with other compounds which enhance the therapeutic agent's effect. For example, a vasoactive compound, such as epinephrine, could be co-administered to restrict local blood flow, and, thus, reduce diffusion of the therapeutic agent away from the injection site.

If desired, the in vivo stability of the collagen formulation following injection can be increased by increasing its resistance to digestion and degradation. This is achieved by increasing the crosslinking in the collagen molecules by either adding a fibrillar component to the collagen solution or by chemically inducing a crosslinking reaction. A therapeutic agent can also be chemically bound and crosslinked to the collagen, in eithersoluble, partially soluble, or intact fibrillar form. Both collagen-collagen crosslinks and therapeutic agent-collagen crosslinks effectively slow the release of the therapeutic agent, and, thus, extend the duration of delivery.

Any appropriate fibrillar component may be utilized to increase the in vivo stability of the collagen. For example, fibrillar collagen may be reconstituted collagen prepared from animal sources, such as bovine hide, using methods described in Borel and Randoux (Frontiers in Matrix Biology, Vol. 10, pages 1-58, In Methods of Connective Tissue Research, Eds. Robert, Moczar, and Moczar, S. Karger, Basel, 1985). Fibrillar collagen may also be prepared from human or animal tissue sources using methods described in Kelman and DeVore (U.S. Patent Nos. 4,969,912 and 5,322,802). The concentrations of the fibrillar collagen component may range between 0.1-2.0% fibrillar collagen solids (w/v).

There are many potential chemical "stabilizing" compounds for use in chemically cross-linking the collagen molecules and the therapeutic agents, e.g., crosslinking collagen amine groups together or crosslinking collagen amine groups to amine, carboxyl, phenol, sulfonyl, or carbohydrate groups of therapeutic agents. Suitable crosslinking compounds include bifunctional acylation agents, including anhydrides, acid chlorides, sulfonyl chlorides, e.g., 1 ,2,3,4-cyclobutanetetracarboxylic dianhydride, tetrahydrofuran-2,3,4,5-tetracarboxylic dianhydride, 1,2,4,5-benzenetetracarboxylic di-anhydride, ethylenediaminetetraacetic dianhydride, bicyclo(2,2,2)oct-7-ene-2,3,5,6-tetracarboxylic dianhydride, glutaryl dichloride, adipoyl chloride, 3-methyladipoyl chloride, pimeloyl chloride, terephthaloyl chloride, isophthaloyol dichloride, phthaloyl dichloride, 1 ,4-phenylene bis(chloroformate), 2,4-mesitylenedisulfonyl chloride, 2,6-naphthalenedisulfonyl chloride, malonyl dichloride, homobifunctional amine cross-reactive cross-linkers, such as homobifunctional imidoesters and homobifunctional N-hydroxysuccinimidyl, and heterobi functional crosslinkers (Pierce, Rockford, IL).

The collagen-therapeutic agent delivery system described herein is ideally suited to the treatment of hard tissue defects, including bone and cartilage repair, and soft tissue defects, including tendon, ligament, and dermal repair. Such defects can result from injury, degeneration, or from disease. The therapeutic agents administered to enhance repair include the following: for bone repair, mesenchymal stem cells, osteoprogenitor cells, and growth factors which either stimulate bone synthesis or inhibit its resorption, e.g., basic and acidic fibroblast growth factor, insulin-like growth factors I and II, and transforming growth factor beta; for cartilage repair, mesenchymal stem cells, chondrocytes, growth factors, cytokines, and angiogenic factors; for tendon and ligament repair, mesenchymal stem cells; and for general soft tissue repair and wound healing (e.g., in connective tissue, neural tissue, organs, or vasculature tissue), a promoter of fibroblast infiltration, a fibroblast cell preparation, and a promoter of collagen secretion, angiogenesis, or wound closure (e.g., PDGF-A, PDGF-B, FGF, VEGF, and leptin.) The disclosed collagen delivery system may also be used for periodontal and anti-inflammatory treatments.

In another application, the collagen-therapeutic agent delivery system described herein is also suited to administer a therapeutic agent to inhibit cell proliferation. Such inhibition is beneficial, for example, in the treatment of cancer. The therapeutic agent is administered at the rumor or at the site of tumor resection to inhibit cancer cell proliferation.

Examples of cell proliferation inhibitors that are used as therapeutic agents are as follows: anti-metabolic antibiotics, e.g., mitomycin, daunorubicin, mithramycin, bleomycin, or doxorubicin; anti-metabolites, e.g., 5-fluorouracil, 5-fluorouridine-5'-monophosphate, 5-fluorodeoxyuridine, 5-fluorodeoxyuridine-5'-monophosphate, and 5-fluoroorotate; anti-fibrotics, such as inhibitors of prolyl hydroxylase and lysyl hydroxylase (e.g., iron chelators, , -dipyridyl, o-phenanthroline, proline analogs, lysine analogs, and free radical inhibitors and scavengers), inhibitors of collagen secretion (e.g., colchicine, vinblastin, cytochalasin B, copper, zinc, and EGTA), inhibitors of collagen secretion and maturation (e.g., BAPN, vincristine, and D-penicillamine); stimulators of collagen degradation, e.g., EDTA and colchicine; anti-viral compounds, e.g., vidarabine, acyclovir, AZT, and amantadine; angiostatic compounds, e.g., angiostatin; and other miscellaneous anti-cell proliferative drugs, e.g., tissue plasminogen activator (TPA), heparin, cytosine arabinoside, and gamma-interferon.

In addition, it may be desirable to administer a collagen-antibiotic solution either as the sole therapeutic agent in patients at risk of developing local infection, e.g., post-operative and diabetic patients, or in combination with another therapeutic agent. Similarly, an antiviral compound can be administered, e.g., injection of a collagen-acyclovir formulation could provide a sustained release system for treating local outbreaks of herpes infection.

There is described below one set of experiments demonstrating that the present collagen delivery system provides a means by which cells may be delivered, for example, to a bone, cartilage, meniscus, tendon, ligament, or dermal defect, to promote healing through the stimulation of new matrix formation.

An additional set of experiments demonstrates that the present collagen delivery system may be used to deliver various therapeutic agents. The characteristics of the collagen formulation can be modified to allow for extending the duration of therapeutic agent release, if desired, by increasing the crosslinking within the collagen formulation and by crosslinking the therapeutic agent to the collagen.

These examples are provided for the purpose of illustrating the invention and should not be construed as limiting.

### A Rapidly Polymerizing Collagen-Mesenchymal Stem Cell Preparation

Samples of collagen were prepared from rabbit dermis by the methods described in DeVore and Eiferman (U.S. Patent No. 5,492,135). The soluble collagen, formulated at either 10 or 20 mg/ml, was then mixed with 7.5 million rabbit mesenchymal stem cells (MSC). The collagen-MSC solution was then either (1) incubated for three weeks, during which time gelation occuned spontaneously, (2) polymerized to form a gel, incubated for 7 days, and then implanted into nude mice subcutaneously, or (3) immediately injected into nude mice subcutaneously. All implants were removed 3-6 weeks post-implantation and all collagen-MSC gels were examined histologically.

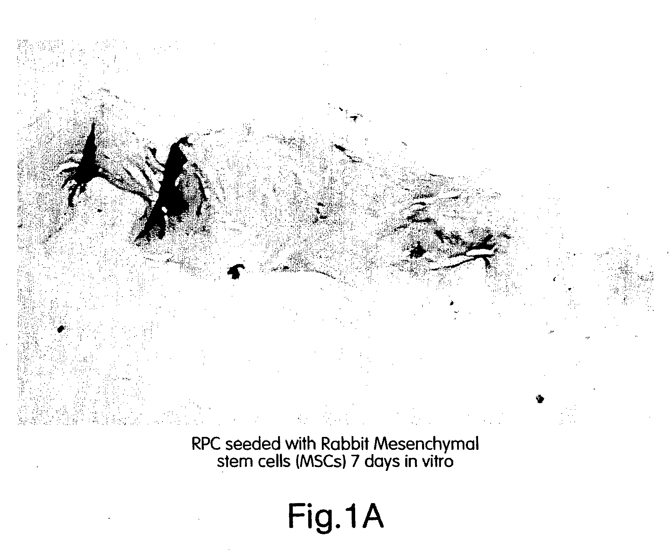

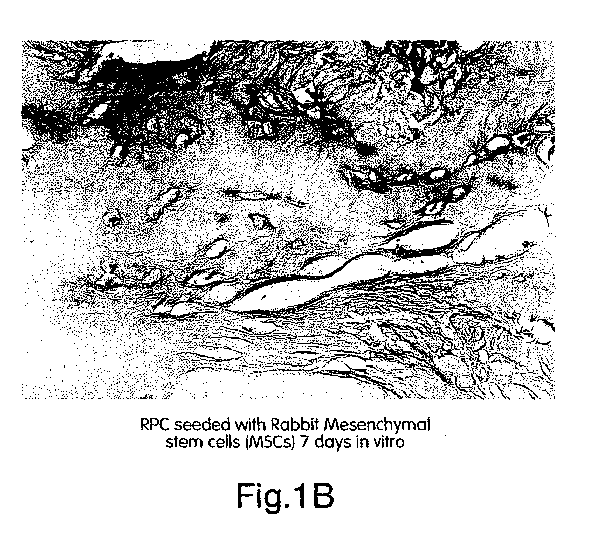

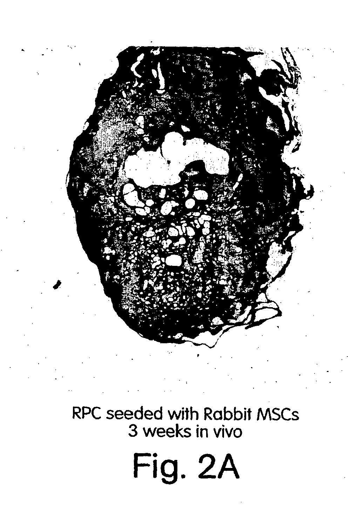

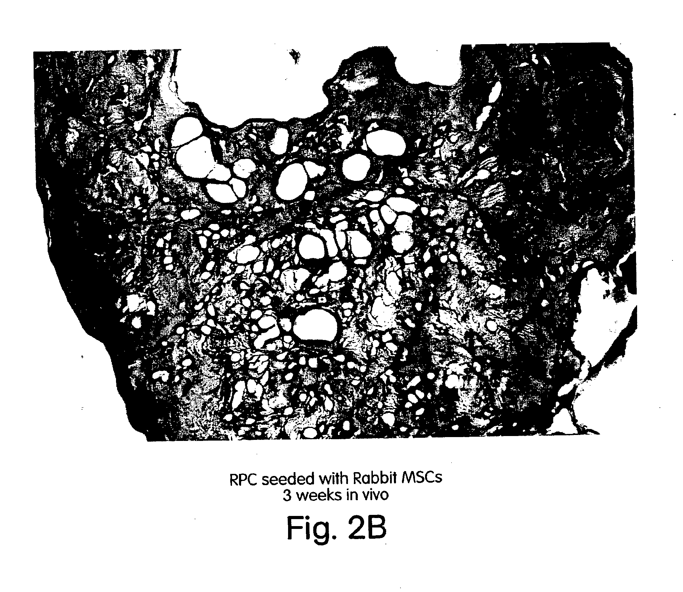

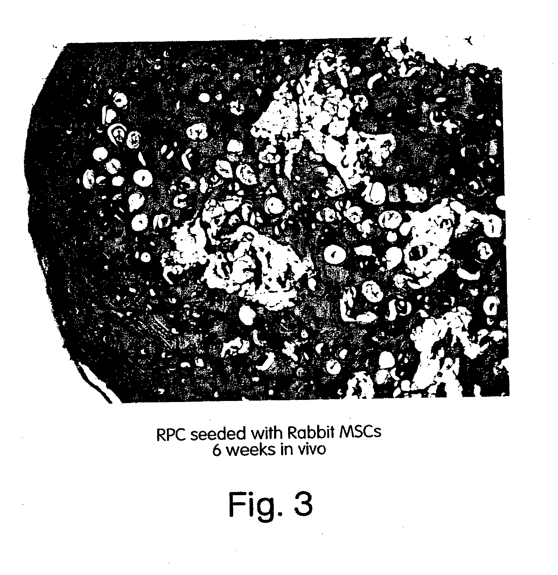

Collagen-MSC gels incubated in vitro for 7 days showed no matrix formation (Figs. 1A-B). In contrast, collagen-MSC gels removed from the mice demonstrated chondrocyte-like cell and metachromatic matrix formation, whether the gels were pre-polymerized and implanted, or polymerized after injection into mice. In pre-polymerized gels, chondrocyte hypertrophy and endochondral bone formation were observed after removal 3 weeks post-implantation (Figs. 2A-B). In gels polymerized after injection, an even more robust chondrogenesis and osteogenesis response was observed after removal 6 weeks post-injection. These gels also exhibited the formation of openings resembling marrow space (Fig. 3).

These results clearly demonstrated the ability of the rapidly polymerizing collagen formulation to deliver mesenchymal stem cells for enhancement of new matrix formation. In addition, all injection sites "set-up" into gelatinous-fibrous plugs with no indication of diffusion or migration. These results indicated that this system is ideal for injectable delivery of mesenchymal stem cells to treat bone, cartilage, tendon, and ligament defects, as well as the delivery of other therapeutic agents to other specific sites.

### Binding and Release of a Therapeutic Agent from a Collagen Preparation

### Release of demeclocycline and fluorescamine from collagen gels

Samples of collagen were prepared from rabbit dermis by the methods described in DeVore and Eiferman (U.S. Patent No. 5,492,135). Briefly, type I collagen was extracted from calf hide, purified, and acid solubilized in 0.1 N acetic acid, dialyzed extensively, and the pH adjusted to 7.5. To prepare the therapeutic agent-collagen samples, fluorescamine (1 mg/ml) or demeclocycline (15 mg/ml), in combination with a 5% coupling agent, was added to the collagen preparation and allowed to react (two hours for fluorescamine or 30 minutes for demeclocycline). The fluorescamine-collagen solution was then dialyzed against deionized water for 24 hours.

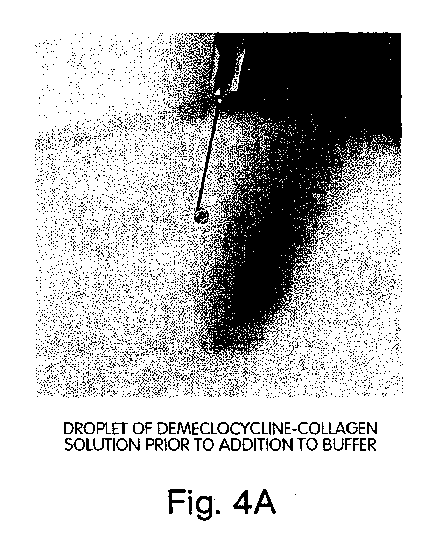

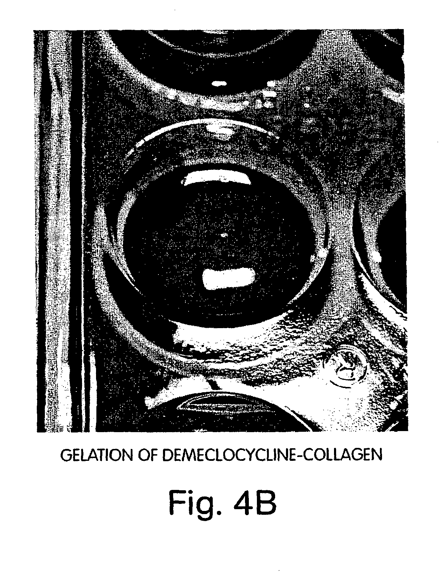

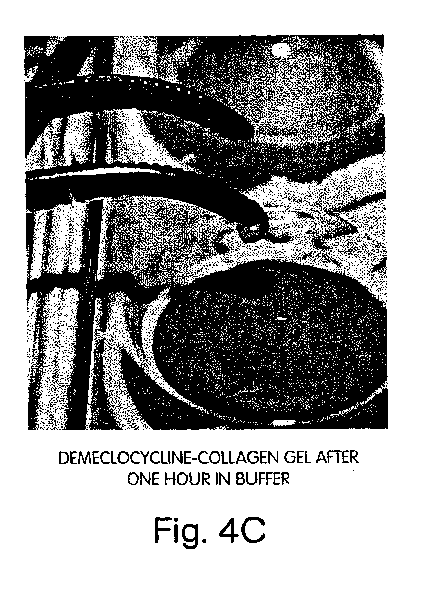

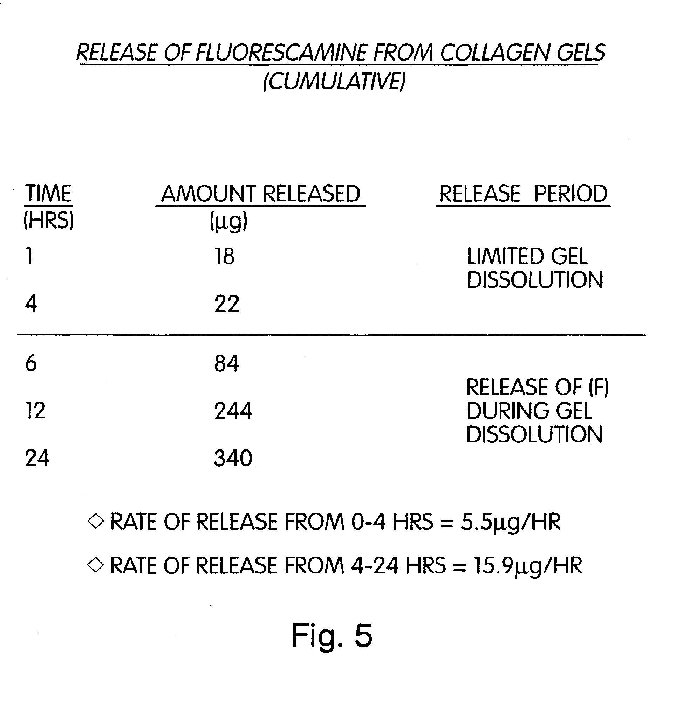

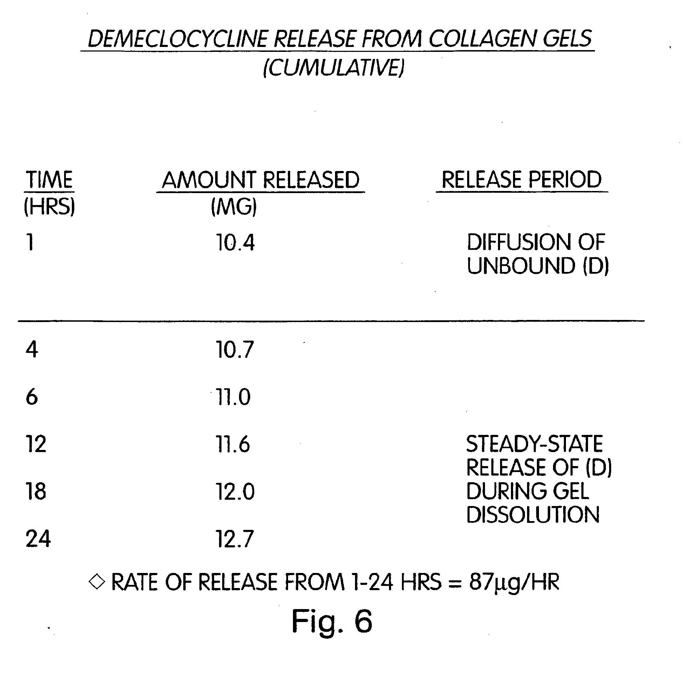

Both the fluorescamine- and demeclocycline-collagen solutions were formed rapidly into gels upon exposure to phospate buffered saline (Fig. 4). The gel pellets were gently agitated in buffer at 37 °C. Aliquots of buffer were removed at various time points up to 54 hours, showing increasing concentrations of collagen and either fluorescamine (Fig. 5) or demeclocycline (Fig. 6), indicating that the agents were released as the pellets dissolved in the buffer.

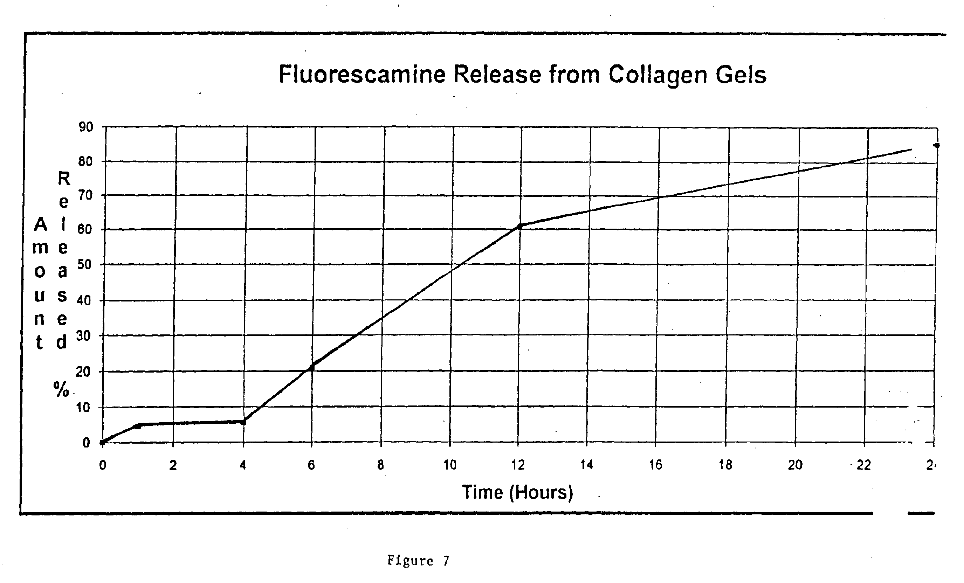

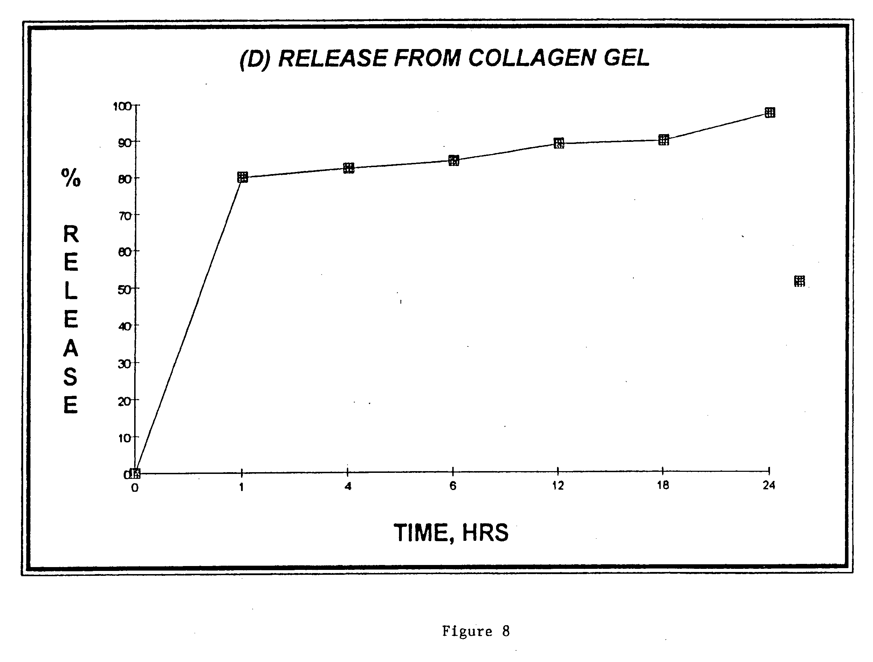

These results demonstrate that the rapidly gelling collagen preparations provide a unique vehicle for local delivery of a therapeutic agent. The rapidly gelling aspect allows the collagen to set-up in a particular injection site and release the therapeutic agent at the site over a period of time. The duration of release may be a function of gel dissolution (Figs. 7 and 8).

### Release of acyclovir from collagen gels, collagen films, and fibrillar collagen preparations

To assay the binding and release of acyclovir from various collagen preparations, rabbit or human dermis was minced, stirred in 70% alcohol for at least 2 hours, stirred in 0.1 N hydrochloric acid for at least 2 hours, and then submersed in 0.5 M Tris buffer, pH 9.0, for at least 2 more hours. The tissue was homogenized several times using a Waring blender, and acyclovir (Zovirax, 10 mg/ml, Burroughs Wellcome Co., Research Triangle Park, NC) was added. To this tissue homogenate, the bifunctional anhydride 3,3 ',4,4'-benzophene tetracarboxylic dianhydride (1 :20 parts tissue) was added, followed by dispersion using glutaric anhydride (1 : 10 parts tissue). The tissue suspension was re-homogenized. After 30 minutes, an additional aliquot of glutaric anhydride (1 : 10 parts tissue) was added, and the tissue suspension was homogenized.

Following centrifugation (8,000 rpm for 20 minutes), the treated tissue suspension separated into a clear, gelatinous collagen fraction, a soluble collagen fraction, a fraction of fine, dispersed tissue containing fibrillar collagen, and particulate residue. The soluble fraction and the dispersed fibrillar collagen fractions were washed and stored in 70% alcohol. The gelatinous fraction was placed in saline solution (0.9%).

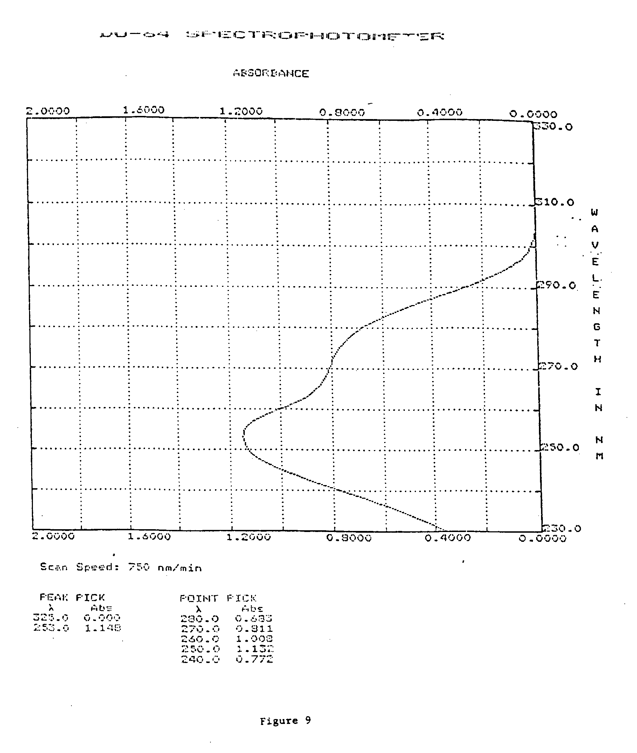

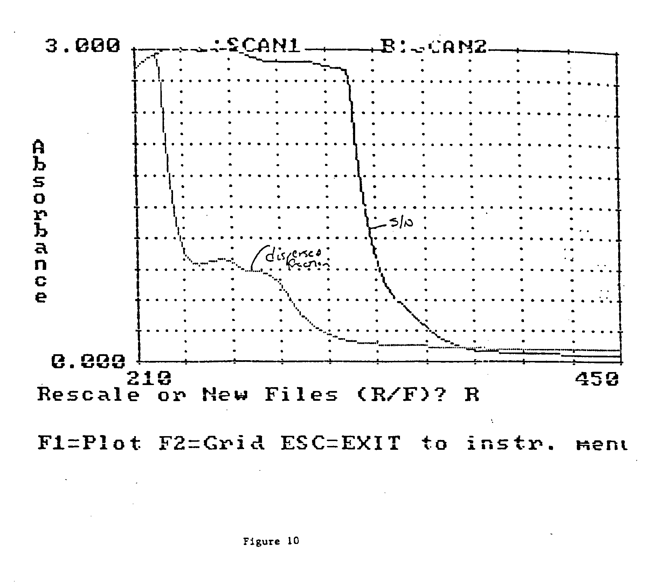

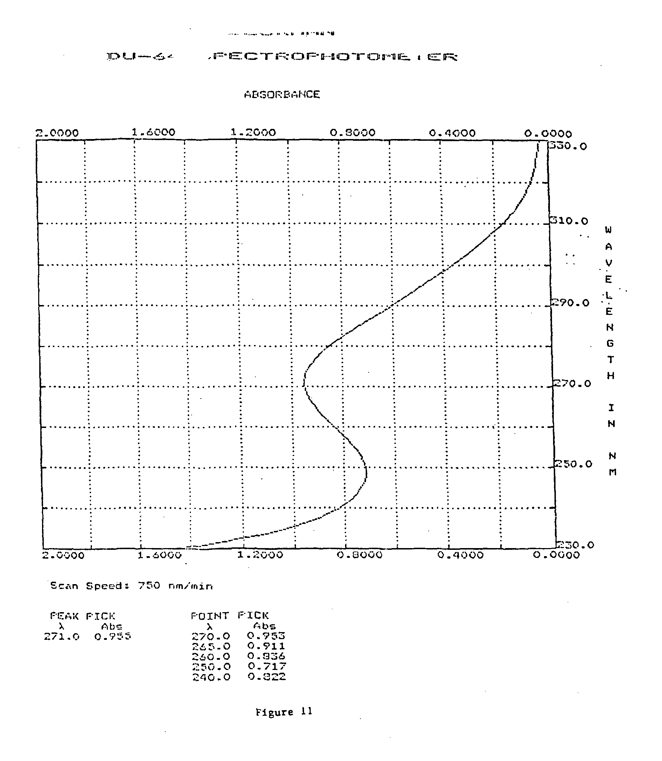

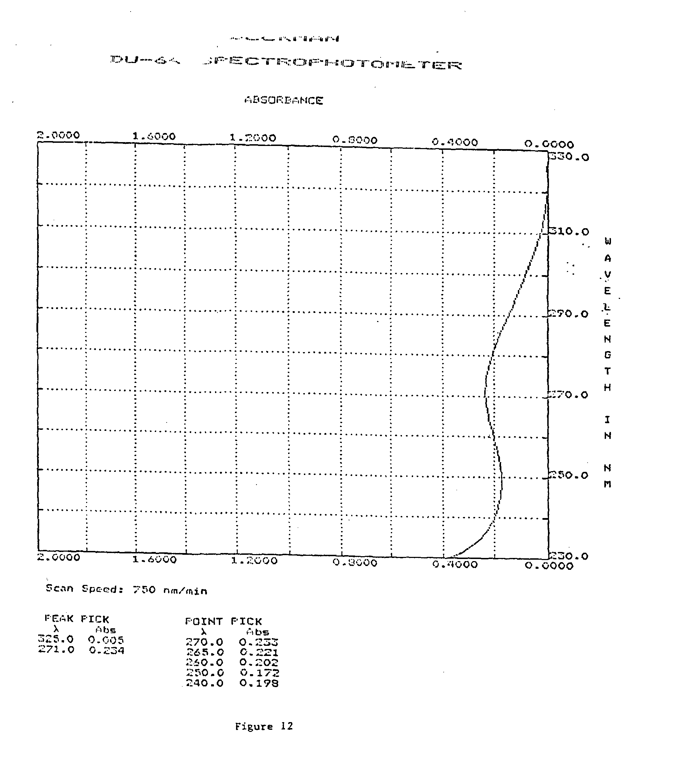

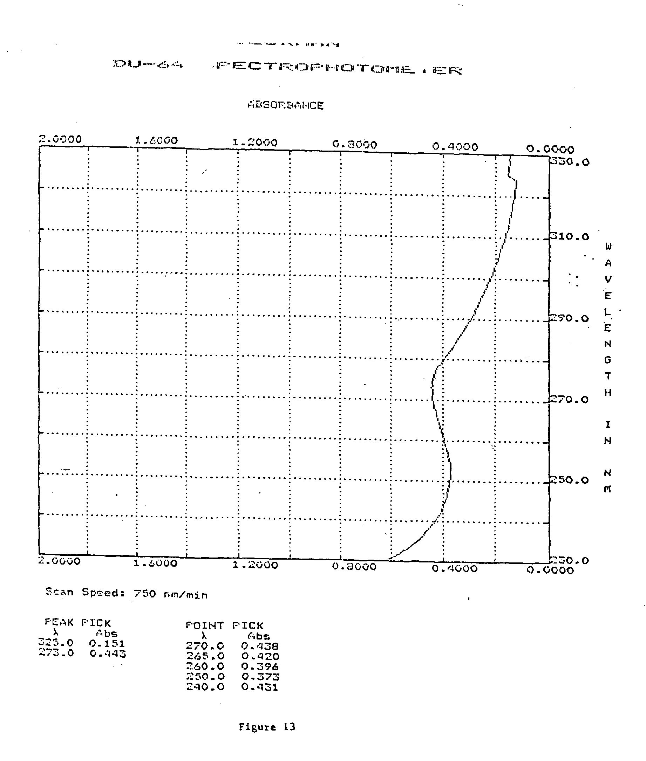

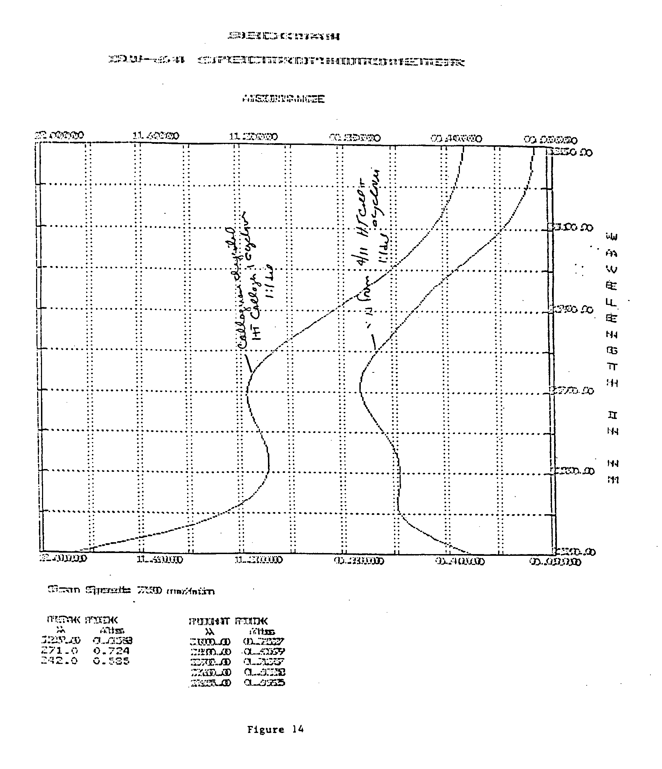

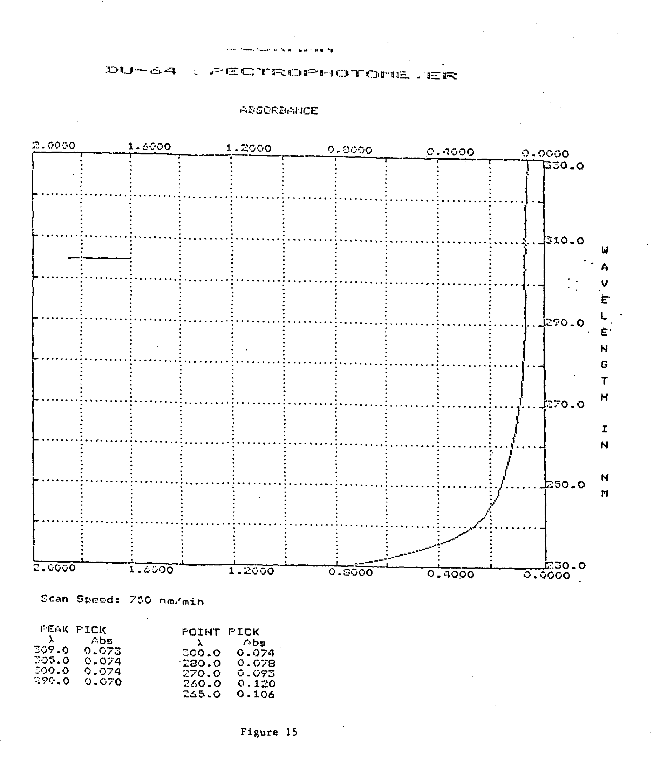

The collagen fractions were analyzed spectrophotometrically to determine acyclovir binding. Peak absorbance for acyclovir occurs at about 250 nM and 270 nM (Fig. 9). Acyclovir was present in the rabbit (Fig. 10) and human (Fig. 11) fibrillar collagen fractions, demonstrating acyclovir binding. Acyclovir binding in the human fibrillar fraction was demonstrated 8 days after trypsin treatment. No acyclovir appeared in the trypsin supernatant. After 24 days of trypsin treatment, the human fibrillar fraction was washed in PBS. Figures 12 and 13 show that some acyclovir was released in the first and second PBS washes, respectively (with the 270 nM acyclovir peak being diluted in the second wash). The PBS-washed human fibrillar fraction was then digested with bacterial collagenase. As shown in Figure 14, acyclovir was still bound to the human fibrillar fraction following 24 days of trypsin treatment and was released upon collagenase digestion, showing a typical acyclovir absorbance profile. No acyclovir absorbance peak was present in the control, non-acyclovir treated fibrillar fraction, as shown in Figure 15.

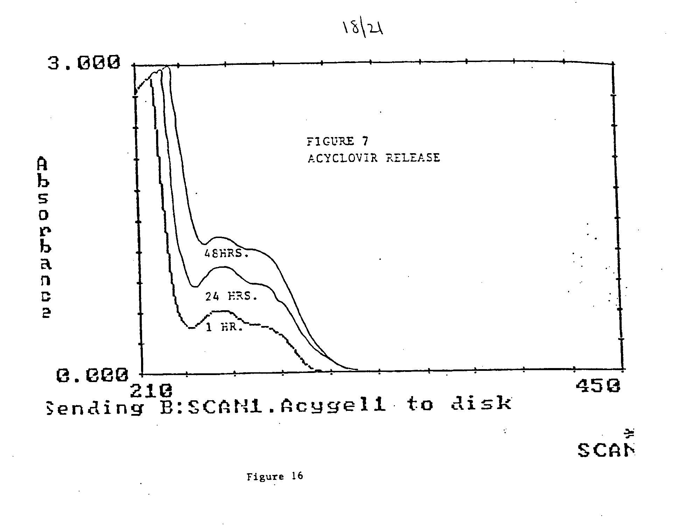

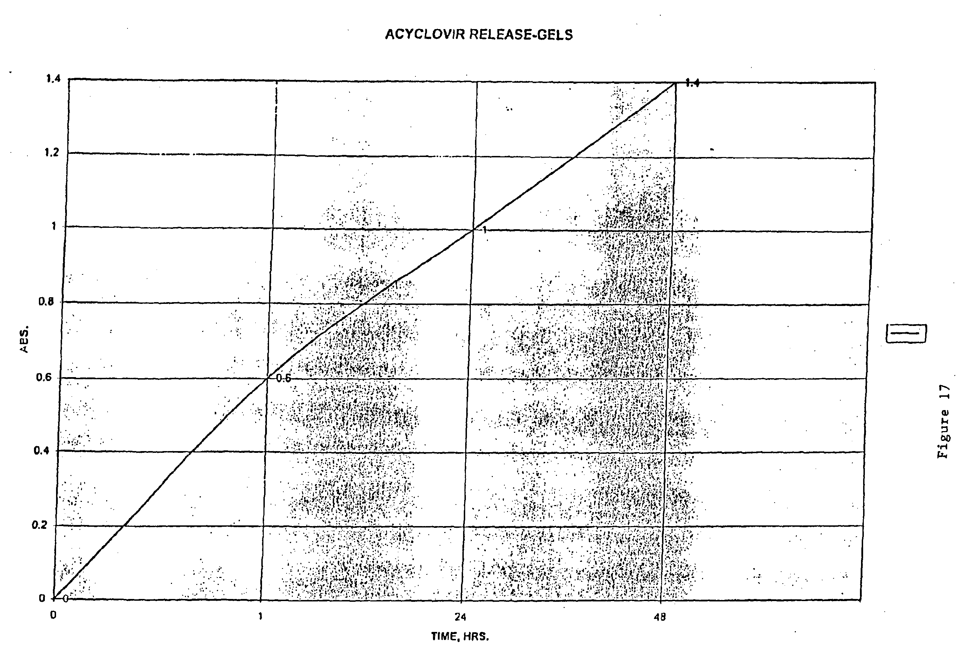

The gelatinous acyclovir fraction was placed in saline solution (0.9%) and saline aliquots were scanned spectrophotometrically to determine release of acyclovir over time. Figures 16 and 17 show the release of acyclovir from the collagen-based gels from 1 to 48 hours. The results demonstrated that acyclovir was released continuously for at least 48 hours. The soluble fraction was also formed into a collagen-acyclovir film.

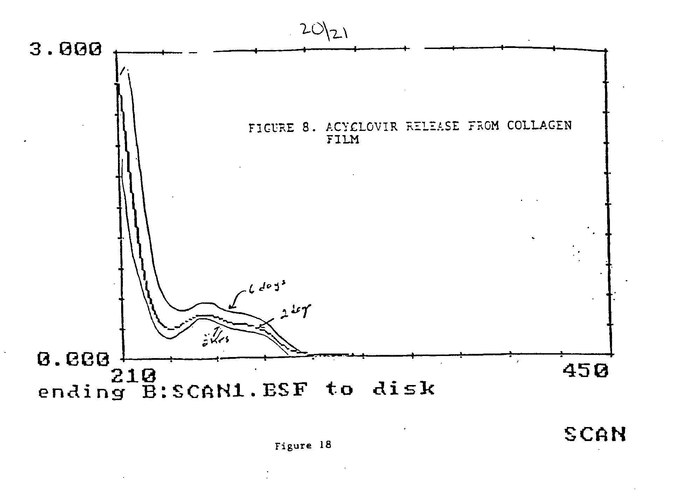

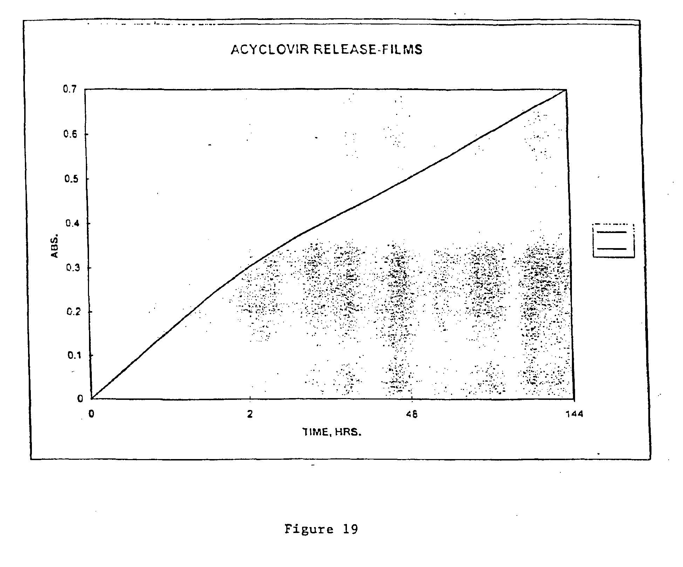

The collagen solution was pipetted into a ceramic mold and air dried in the laminar flow hood. After 24 hours, the film was subjected to ultraviolet irradiation (to crosslink the collagen molecules) in nitrogen for 20 minutes. The film was placed in 0.9% saline solution and incubated at 37 °C. Aliquots were taken for spectrophotometric evaluation. Figures 18 and 19 demonstrate acyclovir binding and the slow release kinetics from the collagen-based films. After 6 days, the film was still intact.

These experiments demonstrate that acyclovir can be chemically bound to fibrillar, gelatinous, and soluble collagen, and released as the collagen is enzymatically digested, ranging in duration from less than 1 hour to more that 28 days. Presumably, the release of the therapeutic agent continues until the collagen is completely dissolved. Collagen digestion was most rapid in the gel formulation (which contained no fibrillar component), intermediate in the film, and slowest in the fibrillar fraction. Therefore, given that the rate of collagen digestion and resorption can be somewhat controlled by the degree of crosslinking (acylation) in the collagen formulation, the rate of therapeutic agent release and the duration of delivery can also be controlled by choosing the appropriate collagen formulation to be used in the injectable delivery system.

## Claims

1. Initially soluble collagen capable of rapid polymerization for use as a vehicle for delivering stem cells, wherein said stem cells are combined with the soluble collagen to form a stem cell-collagen solution which after administration to a tissue site and upon contact with said tissue rapidly polymerizes to a gel, thereby delivering said stem cells to the tissue site, wherein the collagen solution further comprises a fibrillar collagen component,
wherein upon contact with a tissue said solution is converted to a gel within time intervals selected from the group of 180 seconds, 120 seconds, and 90 seconds, and
wherein said fibrillar collagen component is at a concentration of between 0.01-2.0% fibrillar collagen solids (w/v).

2. The initially soluble collagen for use according to claim 1, wherein said stem cells are mesenchymal stem cells.

3. The initially soluble collagen for use according to any of claims 1 to 2, wherein said solution is injectable.

4. The initially soluble collagen for use according to any of claims 1 to 3, wherein said tissue site is bone, cartilage, meniscus, tendon, ligament, or dermis.

5. The initially soluble collagen for use according to any of claims 1 to 4, wherein said tissue site has a defect.

6. The initially soluble collagen for use according to claim 5, wherein said defect is treated by said delivering of said stem cells to said tissue site.

7. The initially soluble collagen for use according to any of claims 1-6, wherein the duration of delivering of said therapeutic agent or said cells is extended by crosslinking said therapeutic agent to said collagen in said solution or by increasing the crosslinking of said collagen in said solution.

8. The initially soluble collagen for use according to claim 7, wherein said crosslinking is induced chemically or by UV radiation.

9. A stem cell-collagen solution capable of rapidly polymerizing to a collagen gel upon contact with a tissue, wherein the collagen solution further comprises a fibrillar collagen component,
wherein upon contact with a tissue said solution is converted to a collagen gel within time intervals selected from the group of 180 seconds, 120 seconds, and 90 seconds, and
wherein said fibrillar component is at a concentration of between 0.01-2.0% fibrillar collagen solids (w/v).

10. The solution according to claim 9, wherein said stem cells are mesenchymal stem cells.

11. The solution according to any of claims 9 to 10, wherein said solution is injectable.

12. The solution according to any of claims 9 to 11, wherein said stem cells are crosslinked to said collagen in said solution.

13. The solution according to claim 12, wherein said collagen is crosslinked in said solution.

14. The solution according to claim 12 or 13, wherein said crosslinking is induced chemically or by UV radiation.

## Patentansprüche

1. Anfänglich lösliches Kollagen, das schnell polymerisieren kann, zur Verwendung als Träger zur Lieferung von Stammzellen, worin die Stammzellen mit dem löslichen Kollagen unter Bildung einer Stammzellen-Kollagen-Lösung kombiniert werden, die nach Verabreichung an eine Gewebestelle und beim Kontakt mit dem Gewebe schnell zu einem Gel polymerisiert, wodurch die Stammzellen zu der Gewebestelle geliefert werden, worin die Kollagenlösung weiter eine fibrilläre Kollagenkomponente umfasst,
worin beim Kontakt mit einem Gewebe die Lösung innerhalb eines Zeitintervalls ausgewählt aus der Gruppe von 180 Sekunden, 120 Sekunden und 90 Sekunden zu einem Gel umgewandelt wird, und
worin die fibrilläre Kollagenkomponente in einer Konzentration von zwischen 0,01-2 % fibrilläre Kollagenfeststoffe (Gew./Vol.) vorliegt.

2. Anfänglich lösliches Kollagen zur Verwendung nach Anspruch 1, worin die Stammzellen mesenchymale Stammzellen sind,

3. Anfänglich lösliches Kollagen zur Verwendung nach einem der Ansprüche 1 bis 2, worin Lösung injizierbar ist.

4. Anfänglich lösliches Kollagen zur Verwendung nach einem der Ansprüche 1 bis 3, worin die Gewebestelle Knochen, Knorpel, Meniskus, Sehne, Ligament oder Haut ist.

5. Anfänglich lösliches Kollagen zur Verwendung nach einem der Ansprüche 1 bis 4, worin die Gewebestelle eine Verletzung aufweist.

6. Anfänglich lösliches Kollagen zur Verwendung nach Anspruch 5, worin die Verletzung durch Liefern der Stammzellen zu der Gewebestelle behandelt wird.

7. Anfänglich lösliches Kollagen zur Verwendung nach einem der Ansprüche 1 - 6, worin die Dauer der Lieferung des therapeutischen Mittels oder der Zellen verlängert wird durch Vernetzen des therapeutischen Mittels mit dem Kollagen in der Lösung oder durch Steigern der Vernetzung des Kollagens in der Lösung

8. Anfänglich lösliches Kollagen zur Verwendung nach Anspruch 7, worin die Vernetzung chemisch oder durch UV-Bestrahlung initiiert wird.

9. Stammzellen-Kollagen-Lösung, die beim Kontakt mit einem Gewebe schnell zu einem Kollagengel polymerisieren kann, worin die Kollagenlösung weiter eine fibrilläre Kollagenkomponente umfasst,
worin beim Kontakt mit einem Gewebe die Lösung innerhalb eines Zeitintervalls ausgewählt aus der Gruppe von 180 Sekunden, 120 Sekunden und 90 Sekunden zu einem Kollagengel umgewandelt wird, und
worin die fibrilläre Kollagenkomponente in einer Konzentration von zwischen 0,01-2 % fibrilläre Kollagenfeststoffe (Gew./Vol.) vorliegt.

10. Lösung nach Anspruch 9, worin die Stammzellen mesenchymale Stammzellen sind.

11. Lösung nach einem der Ansprüche 9 bis 10, worin Lösung injizierbar ist.

12. Lösung nach einem der Ansprüche 9 bis 11, worin die Stammzellen mit dem Kollagen in der Lösung vernetzt werden.

13. Lösung nach Anspruch 12, worin das Kollagen in der Lösung vernetzt wird.

14. Lösung nach Anspruch 12 oder 13, worin die Vernetzung chemisch oder durch UV-Bestrahlung initiiert wird.

## Revendications

1. Collagène initialement soluble capable d'une polymérisation rapide pour une utilisation comme véhicule pour délivrer des cellules souches, dans lequel lesdites cellules souches sont combinées avec le collagène soluble pour former une solution de cellules souches-collagène qui, après administration à un site tissulaire et lors du contact avec ledit tissu polymérise rapidement en un gel, délivrant de ce fait lesdites cellules souches au site tissulaire, dans lequel la solution de collagène comprend en outre un composant de collagène fibrillaire,
dans lequel lors du contact avec un tissu, ladite solution est convertie en un gel dans des intervalles de temps choisis dans le groupe de 180 secondes, 120 secondes, et 90 secondes, et
dans lequel ledit composant de collagène fibrillaire est à une concentration comprise entre 0,01 et 2,0 % de matières solides de Collagène fibrillaire (p/v).

2. Collagène initialement soluble pour une utilisation selon la revendication 1, dans lequel lesdites cellules souches sont des cellules souches mésenchymateuses.

3. Collagène initialement soluble pour une utilisation selon l'une quelconque des revendications 1 à 2, dans lequel ladite solution est injectable.

4. Collagène initialement soluble pour une utilisation selon l'une quelconque des revendications 1 à 3, dans lequel ledit site tissulaire est un os, un cartilage, un ménisque, un tendon, un ligament, ou le derme.

5. Collagène initialement soluble pour une utilisation selon l'une quelconque des revendications 1 à 4, dans lequel ledit site tissulaire présente un défaut.

6. Collagène initialement soluble pour une utilisation selon la revendication 5, dans lequel ledit défaut est traité par ladite délivrance desdites cellules souches audit site tissulaire.

7. Collagène initialement soluble pour une utilisation selon l'une quelconque des revendications 1 à 6, dans lequel la durée de délivrance dudit agent thérapeutique ou desdites cellules est prolongée par la réticulation dudit agent thérapeutique audit collagène dans ladite solution ou par l'augmentation de la réticulation dudit collagène dans ladite solution.

8. Collagène initialement soluble pour une utilisation selon la revendication 7, dans lequel ladite réticulation est induite chimiquement ou par rayonnement UV.

9. Solution de cellules souches-collagène capable de polymériser rapidement en un gel de collagène lors du contact avec un tissu, dans laquelle la solution de collagène comprend en outre un composant de collagène fibrillaire,
dans laquelle lors du contact avec un tissu, ladite solution est convertie en un gel de collagène dans des intervalles de temps choisis dans le groupe de 180 secondes, 120 secondes, et 90 secondes, et
dans laquelle ledit composant fibrillaire est à une concentration comprise entre 0,01 et 2,0 % de matières solides de collagène fibrillaire (p/v).

10. Solution selon la revendication 9, dans laquelle lesdites cellules souches sont des cellules souches mésenchymateuses.

11. solution selon l'une quelconque des revendications 9 à 10, dans laquelle ladite solution est injectable.

12. Solution selon l'une quelconque des revendications 9 à 11, dans laquelle lesdites cellules souches sont réticulées en ledit collagène dans ladite solution.

13. Solution selon la revendication 12, dans laquelle ledit Collagène est réticulé dans ladite solution.

14. Solution selon la revendication 12 ou 13, dans laquelle ladite réticulation est induite chimiquement ou par rayonnement UV.
